# EUROPEAN PATENT APPLICATION

(11) **EP 4 197 467 A1**
(43) Date of publication of application: **21.06.2023**
(21) Application number: 22214106.1
(22) Date of filing: 16.12.2022
(51) Int. Cl.: A61B 18/00, A61B 18/14, A61B 17/00, A61B 34/20

(54) **HIGH-FREQUENCY TISSUE ABLATION USING COATED ELECTRODES**

(30) Priority: 17.12.2021 US 202163291288 P; 23.11.2022 US 202218058579
(71) Applicant: Biosense Webster (Israel) Ltd, 2066717 Yokneam (IL)
(72) Inventor: Govari, Assaf, Yokneam 2066717 (IL); Beeckler, Christopher Thomas, Irvine, CA 92618 (US)
(74) Representative: Carpmaels & Ransford LLP

(57) **Abstract**

A method for fabricating a medical device includes providing a metal electrode to be used in applying electrical energy to biological tissue and specifying a frequency at which the electrical energy is to be applied. A thickness of a ceramic coating to be applied to the metal electrode is identified so as to reduce an electrical impedance between the metal electrode and the tissue at the specified frequency by a specified amount. The ceramic coating is deposited over the metal electrode to the identified thickness, and the metal electrode is assembled onto a probe for application to the biological tissue.

## Description

### FIELD

The present disclosure relates generally to invasive medical equipment and procedures, and particularly to apparatus and methods for ablating tissue within the body.

### BACKGROUND

Irreversible electroporation (IRE) is a soft tissue ablation technique that applies short pulses of strong electrical fields to create permanent and hence lethal nanopores in the cell membrane, thus disrupting the cellular homeostasis (internal physical and chemical conditions). Cell death following IRE results from apoptosis (programmed cell death) and not necrosis (cell injury, which results in the destruction of a cell through the action of its own enzymes) as in other thermal and radiation-based ablation techniques. IRE may be used in tumor ablation, particularly in regions where precision and conservation of the extracellular matrix, blood flow and nerves are of importance.

IRE may also be applied in ablating tissue in the heart, for example as described in U.S. Patent Application Publication 2021/0161592, whose disclosure is incorporated herein by reference. As explained in this reference, IRE is a predominantly non-thermal process, which should cause an increase of the tissue temperature by, at most, a few degrees for a short time. It thus differs from RF (radio frequency) ablation, which raises the tissue temperature by between 20°C and 70°C and destroys cells through heating. IRE may utilize biphasic pulses, i.e., combinations of positive and negative pulses, which are applied, for example, between two bipolar electrodes of a catheter. In order for the IRE pulses to generate the required nanopores in heart tissue, the field strength of the pulses must exceed a tissue-dependent threshold Eₜₕ, which for heart cells is approximately 500 V/cm. The applied voltages may reach up to 2000 V. A biphasic IRE pulse comprises a positive and a negative pulse applied between two electrodes with short pulse widths and a brief separation between the positive and negative pulses.

Electrodes on ablation catheters are typically made from biocompatible metals, such as gold or platinum. In some cases a coating is applied over the metal electrode. For example, U.S. Patent 6,475,214 describes an ablation catheter comprising an elongated, flexible catheter body having proximal and distal ends and at least one lumen extending therethrough. A tip electrode having a length of at least about 3 mm is mounted on the distal end of the catheter body. The tip electrode comprises a base material having an outer surface and a porous layer applied over at least a portion of the outer surface of the base material, the porous layer comprising metal nitride, metal oxide, metal carbide, metal carbonitride, carbon, carboxy nitride, or a combination thereof.

### SUMMARY

Examples of the present disclosure that are described hereinbelow provide improved electrodes for use in IRE and methods for fabrication of such electrodes.

An example method for fabricating a medical device can include the following steps executed in various orders and including interleaving steps as understood by a person skilled in the pertinent art. The method can include providing a metal electrode to be used in applying electrical energy to biological tissue; specifying a frequency at which the electrical energy is to be applied; identifying a thickness of a ceramic coating to be applied to the metal electrode that will reduce an electrical impedance between the metal electrode and the tissue at the specified frequency by a specified amount; depositing the ceramic coating over the metal electrode to the identified thickness; and assembling the metal electrode onto a probe for application to the biological tissue.

Applying the electrical energy can include applying biphasic electrical pulses so as to cause irreversible electroporation of the biological tissue.

Assembling the metal electrode can include fixing the metal electrode to a distal end of a catheter for insertion into a heart of a living subject.

Specifying the frequency can include selecting a frequency in excess of 100 kHz.

The ceramic coating can include titanium nitride.

Identifying the thickness can include selecting the thickness within a range between 100 and 10,000 nm.

Depositing the ceramic coating can include applying a process of electrochemical deposition or physical vapor deposition to the metal electrode.

Identifying the thickness can include choosing the thickness of the ceramic coating to reduce the electrical impedance by at least 25% relative to a baseline impedance between the metal electrode and the tissue.

An example system for medical treatment can include a signal generator, a probe, and a ceramic coating. The signal generator can be configured to generate electrical energy at a specified frequency for application to biological tissue. The probe can include a metal electrode which is configured to be applied to the biological tissue and is coupled to receive the electrical energy from the signal generator. The ceramic coating can be disposed over the metal electrode with a thickness selected so as to reduce an electrical impedance between the metal electrode and the tissue at the specified frequency by a specified amount.

The signal generator can be configured to apply biphasic electrical pulses to the metal electrode so as to cause irreversible electroporation of the biological tissue.

The probe can include a catheter configured for insertion into a heart of a living subject.

The specified frequency can be greater than 100 kHz.

The ceramic coating can include titanium nitride.

The thickness of the ceramic coating can be within a range between 100 and 10,000 nm.

The ceramic coating can be applied to the metal electrode by a process of electrochemical deposition or physical vapor deposition.

The thickness of the ceramic coating can be chosen to reduce the electrical impedance by at least 25% relative to a baseline impedance between the metal electrode and the tissue.

### BRIEF DESCRIPTION OF THE DRAWINGS

The present disclosure will be more fully understood from the following detailed description of the examples thereof, taken together with the drawings in which:
Fig. 1 is a schematic pictorial illustration of a system used in an IRE ablation procedure, in accordance with examples of the disclosure;
Fig. 2A is a schematic pictorial illustration of a basket assembly deployed at the distal end of a catheter used in IRE, in accordance with an example of the disclosure;
Fig. 2B is a schematic sectional illustration showing details of an electrode on the basket assembly of Fig. 2A, in accordance with an example of the disclosure; and
Fig. 3 is a flow chart, which schematically illustrates a method for fabrication of a catheter for use in IRE ablation, in accordance with an example of the disclosure.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

As discussed herein, tissue of a "patient," "host," "user," and "subject" can be tissue of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, or any other individual or delivery instrumentation associated with delivery of a multi-electrode catheter for treatments disclosed herein.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE). Ablating or ablation as it relates to the devices and corresponding systems of this disclosure is used throughout this disclosure in reference to ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to IRE ablation schemes describe ablation schemes which differ with respect to electrical current path and electric field distribution. "Bipolar" refers to an IRE ablation scheme utilizing a current path between two electrodes that are both positioned at a treatment site; current density and electric flux density is typically approximately equal at each of the two electrodes. "Unipolar" refers to an IRE ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

As explained above, IRE is an electrical, rather than a thermal process, involving application of high voltages to the target tissue at high frequencies. The effectiveness of the IRE process is sensitive to the impedance between the electrodes and the tissue, including both the resistive and reactive components of the impedance. To optimize the delivery of energy to the tissue and reduce tissue heating, the impedance at the IRE pulse frequency should be minimized.

Examples of the disclosure that are described herein address this need by coating the ablation electrodes with a thin layer of a suitable ceramic material, such as titanium nitride. (Thin films of certain ceramics, including titanium nitride, have low electrical resistivity.) The thickness of the coating is selected according to the frequency of the IRE pulses that are to be applied, so as to minimize the impedance of the electrode at that frequency.

Thus, an example of the present disclosure provides a method for fabricating a medical device, which comprises a probe for application to a biological tissue, such as an intracardiac catheter, with one or more metal electrodes assembled onto the probe. The frequency at which electrical energy is to be applied by the device to the tissue is specified, for example a pulse frequency in excess of 100 kHz for IRE. The thickness of the ceramic coating that is to be applied to the metal electrodes is selected so as to reduce the electrical output impedance between the electrodes and the tissue at the specified frequency by a specified amount, for instance 25%, relative to the baseline electrical impedance, i.e., the impedance between an uncoated metal electrode and the tissue. The desired coating thickness may be computed from first principles or, alternatively or additionally, it may be found empirically by experimental testing. The ceramic coating is then applied over the metal electrode to the identified thickness. The coating may be applied by any suitable process, for example electrochemical deposition or physical vapor deposition (PVD) typically to a thickness between 100 and 10,000 nm.

Although the examples that are described hereinbelow are directed specifically to ablation of myocardial tissue by IRE, the principles of the present disclosure are similarly applicable in coating electrodes for other therapies involving high electrical frequencies and voltages, as well as electrodes used for sensing.

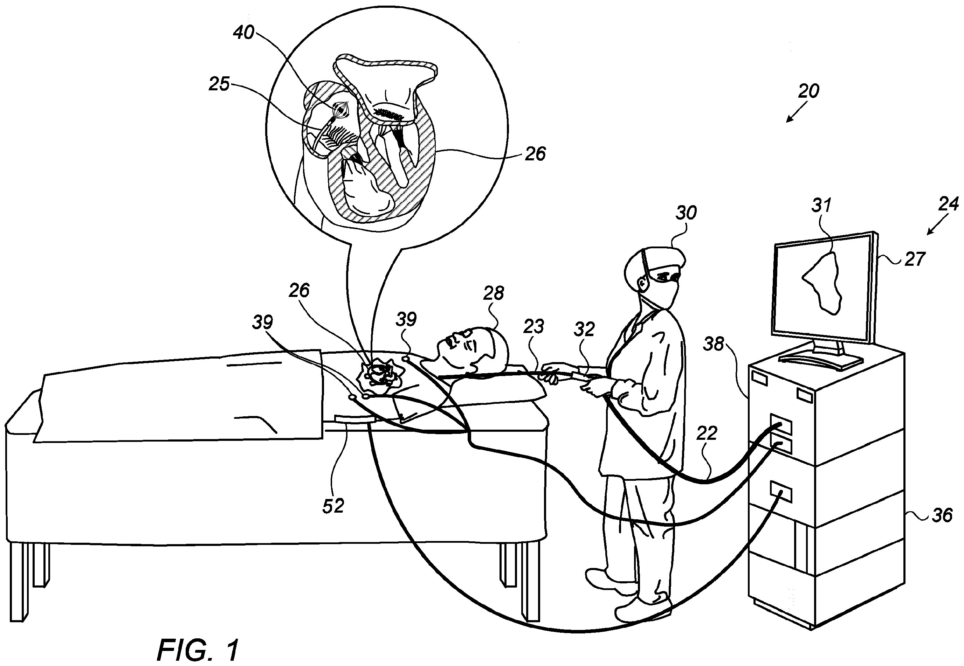

Fig. 1 is a schematic pictorial illustration of a system 20 used in an IRE ablation procedure, in accordance with an example of the disclosure. Elements of system 20 may be based on components of the CARTO^{®} system, produced by Biosense Webster, Inc. (Irvine, California).

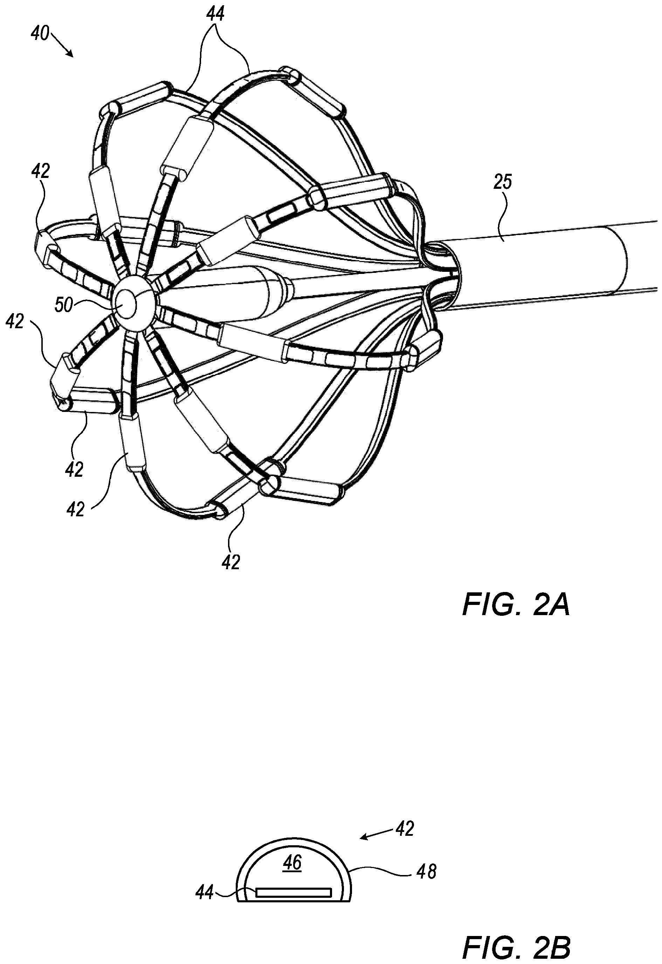

A physician 30 navigates a catheter 22 through the vascular system of a patient 28 into a chamber of a heart 26 of the patient, and then deploys a basket assembly 40 (shown in detail in Fig. 2A) at the distal end of the catheter 22. The proximal end of basket assembly 40 is connected to the distal end of an insertion tube 25, which physician 30 steers using a manipulator 32 near the proximal end of catheter 22. Basket assembly 40 is inserted in a collapsed configuration through a sheath 23, which passes through the vascular system of patient 28 into the heart chamber where the IRE procedure is to be performed, and is then deployed from the sheath and allowed to expand within the chamber. Catheter 22 is connected at its proximal end to a control console 24. A display 27 on console 24 may present a map 31 or other image of the heart chamber with an icon showing the location of basket assembly 40 in order to assist physician 30 in positioning the basket assembly at the target location for the IRE ablation procedure.

The system 20 can include one or more electrode patches 39 positioned for skin contact on the patient 28. The electrode patches 39 may provide impedance-based tracking of electrodes of the basket assembly 40. Details of the impedance-based location tracking technology are described in U.S. Patent Nos. 7,536,218; 7,756,576; 7,848,787; 7,869,865; and 8,456,182 incorporated herein by reference. Additionally, or alternatively, the catheter 22 and/or sheath 23 may include magnetic-based navigation sensors configured to determine a location of the distal end of the catheter 22 and/or sheath 23 within the patient 28. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091 incorporated by reference herein.

Once basket assembly 40 is properly deployed and positioned in heart 26, physician 30 actuates an electrical signal generator 38 in console 24 to apply sequences of IRE pulses to the electrodes on the basket assembly, under the control of a processor 36. The IRE pulses are typically biphasic and applied in a bipolar mode, between pairs of the electrodes on basket assembly 40. Additionally or alternatively, the pulses may be applied between the electrodes on basket assembly 40 and a separate common electrode, for example a conductive back patch 52, which is applied to the patient's skin.

To perform IRE, electrical signal generator 38 typically applies sequences of pulses to the electrodes with an amplitude of at least 200 V, while the duration of each pulse is less than 20 µs. Typically, the pulses include frequencies of at least 100 kHz. The sequences of the pulses comprise biphasic pairs of pulses, meaning that each pair comprises a positive pulse and a negative pulse. (The terms "positive" and "negative" are used arbitrarily to indicate the opposing electrical polarities of the pulses.) The IRE pulse frequency is the frequency at which pulses in an IRE electrical signal are delivered. In the case of biphasic pulses, the IRE frequency is defined by frequency at which each pair of positive and negative pulses are delivered. For biphasic pulses delivered in a pulse train, the IRE pulse frequency is equal to an inverse of a period of a biphasic pulse in the pulse train. An electrical signal generator capable of outputting this sort of IRE pulse sequences, while switching rapidly between different pairs of electrodes, is described, for example, in the above-mentioned U.S. Patent Application Publication No. 2021/0161592 incorporated herein by reference. Examples of various systems and methods of ablation including IRE are presented in U.S. Patent Application Publication Nos. 2021/0169550; 2021/0169567; 2021/0169568; 2021/0196372; 2021/0177503; and 2021/0186604 the entireties of each of which are incorporated herein by reference.

Processor 36 directs electrical signal generator 38 to switch among the electrodes and apply the pulses in accordance with a predefined protocol. Physician 30 may select the protocol to apply using controls on console 24. For example, the physician or an assistant may use a touch screen functionality of display 27 on console 24 to interact with processor 36. Alternatively or additionally, the physician or an assistant may operate the controls in order to select the pulse parameters and electrodes manually.

The system configuration that is shown in Fig. 1 is presented by way of example for conceptual clarity in understanding the operation of examples of the present disclosure. For the sake of simplicity, Fig. 1 shows only certain elements of system 20 that are related to these examples. The remaining elements of the system will be apparent to those skilled in the art, who will likewise understand that the principles of the present disclosure may be implemented in other medical therapeutic systems, using other components. All such alternative implementations are considered to be within the scope of the present disclosure.

Fig. 2A is a schematic pictorial illustration of basket assembly 40, in accordance with an example of the disclosure. Basket assembly 40 comprises multiple resilient spines 44, with electrodes 42 disposed on the spines. In the pictured example, an axial electrode 50 is disposed at the tip of the basket, i.e., at the distal end of the basket assembly. Electrodes 42 and 50 are connected to wires (not shown) running through insertion tube 25 to the proximal end of catheter 22, where they connect to appropriate circuitry in console 24, and specifically to electrical signal generator 38.

Spines 44 typically comprise a suitable, resilient metal or plastic material, for example, and bow radially outward when basket assembly 40 is deployed from sheath 23 into the heart chamber. Although basket assembly 40 is shown as comprising eight spines 44, in alternative examples the basket assembly may comprise a larger or smaller number of spines. Further alternatively, electrodes 42 may be deployed on other sorts of structures at the distal end of a catheter, as well as on the distal portion of the catheter insertion tube itself. In any case, physician 30 manipulates catheter 22 so that electrodes 42 and 50 contact the myocardial tissue at a target location that is to be ablated, and then actuates electrical signal generator 38 to apply electrical energy to the tissue at a specified frequency.

Fig. 2B is a schematic sectional illustration showing details of one of electrodes 42, in accordance with an example of the disclosure. Electrode 42 comprises a metal body 46, comprising a suitable biocompatible metal such as gold or platinum. In the pictured example, metal body 46 contains a lumen, which fits over spine 44, and has an asymmetrical profile, protruding outward away from the center of basket assembly 40. This particular shape is shown by way of example, however, and electrodes of any suitable shape may be used in examples of the present disclosure.

A thin ceramic film 48 is deposited over the outer surface of metal body 46. For example, in some embodiments, film 48 comprises titanium nitride (TiN), which is deposited to a thickness between 100 and 10,000 nm. Alternatively, other conductive ceramic materials may be used, for example iridium oxide (IrOx), with thickness between 100 and 10,000 nm, depending on the specified frequency and other application requirements.

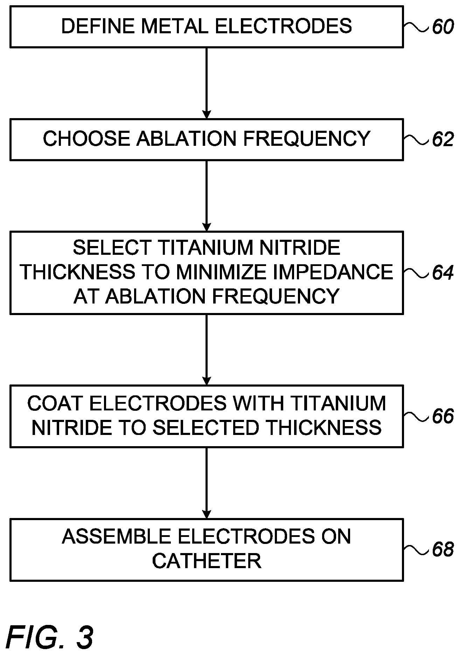

Fig. 3 is a flow chart, which schematically illustrates a method for fabrication of a catheter for use in IRE ablation, in accordance with an example of the disclosure. The method is described, for the sake of convenience and clarity, with reference to the elements of system 20, as shown in Figs. 1, 2A, and 2B. The principles of the present invention, however, may similarly be applied in producing electrodes and medical probes of other sorts.

The characteristics of electrodes 42 are defined, at a metal electrode definition step 60. The definition includes, for example, the type of metal material to be used in metal body 46 of the electrodes and the geometrical shapes and dimensions of the electrodes. An ablation frequency at which the electrodes are to be used is chosen, at a frequency specification step 62.

The thickness of a ceramic coating that is to be applied to metal body 46, such as ceramic film 48, is selected at a film thickness selection step 64. The coating thickness is selected so as to reduce the impedance at the frequency that was chosen at step 62. For example, the thickness is chosen so as to provide a desired reduction, such as 25%, relative to the impedance of the uncoated metal body. Alternatively, the optimal thickness may be selected by a program of trial and error, for example fabricating multiple electrodes with different, respective thicknesses of film 48, followed by testing of the electrodes to measure their impedances at the frequency specified at step 62.

Once the coating thickness has been selected, metal bodies 46 of electrodes 42 are coated with a ceramic material, such as TiN, to the selected thickness, at an electrode coating step 66. Any suitable thin film deposition process can be used for this purpose, for example electrochemical deposition or physical vapor deposition (PVD).

After electrodes 42 have been coated with film 48, they are assembled on a catheter, for example onto basket assembly 40 at the distal end of catheter 22, at an electrode assembly step 68. Once the catheter assembly has been completed, it can be used in system 20, with the ablation frequency tuned to the frequency that was chosen at step 62. Alternatively, uncoated electrodes may be assembled on a catheter, and then the electrodes may be coated after assembly using electrochemical deposition or PVD with the aid of masks.

It will be appreciated that the examples described above are cited by way of example, and that the present invention is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method for fabricating a medical device, the method comprising:
providing a metal electrode to be used in applying electrical energy to biological tissue;
specifying a frequency at which the electrical energy is to be applied;
identifying a thickness of a ceramic coating to be applied to the metal electrode that will reduce an electrical impedance between the metal electrode and the tissue at the specified frequency by a specified amount;
depositing the ceramic coating over the metal electrode to the identified thickness; and
assembling the metal electrode onto a probe for application to the biological tissue.

2. The method according to claim 1, wherein applying the electrical energy comprises applying biphasic electrical pulses so as to cause irreversible electroporation of the biological tissue.

3. The method according to claim 1, wherein assembling the metal electrode comprises fixing the metal electrode to a distal end of a catheter for insertion into a heart of a living subject.

4. The method according to claim 1, wherein specifying the frequency comprises selecting a frequency in excess of 100 kHz.

5. The method according to claim 1, wherein identifying the thickness comprises selecting the thickness within a range between 100 and 10,000 nm.

6. The method according to claim 1, wherein depositing the ceramic coating comprises applying a process of electrochemical deposition or physical vapor deposition to the metal electrode.

7. The method according to claim 1, wherein identifying the thickness comprises choosing the thickness of the ceramic coating to reduce the electrical impedance by at least 25% relative to a baseline impedance between the metal electrode and the tissue.

8. A system for medical treatment, the system comprising:
a signal generator, which is configured to generate electrical energy at a specified frequency for application to biological tissue;
a probe comprising a metal electrode, which is configured to be applied to the biological tissue and is coupled to receive the electrical energy from the signal generator; and
a ceramic coating disposed over the metal electrode with a thickness selected so as to reduce an electrical impedance between the metal electrode and the tissue at the specified frequency by a specified amount.

9. The system according to claim 8, wherein the signal generator is configured to apply biphasic electrical pulses to the metal electrode so as to cause irreversible electroporation of the biological tissue.

10. The system according to claim 8, wherein the probe comprises a catheter configured for insertion into a heart of a living subject.

11. The system according to claim 8, wherein the specified frequency is greater than 100 kHz.

12. The method according to claim 1 or the system according to claim 8, wherein the ceramic coating comprises titanium nitride.

13. The system according to claim 8, wherein the thickness of the ceramic coating is within a range between 100 and 10,000 nm.

14. The system according to claim 8, wherein the ceramic coating is applied to the metal electrode by a process of electrochemical deposition or physical vapor deposition.

15. The system according to claim 8, wherein the thickness of the ceramic coating is chosen to reduce the electrical impedance by at least 25% relative to a baseline impedance between the metal electrode and the tissue.
